# FASCICULE DE BREVET EUROPEEN

(11) **EP 0 424 498 B1**
(45) Date de publication et mention de la délivrance du brevet: **08.06.1994**
(21) Numéro de dépôt: 90907093.0
(22) Date de dépôt: 10.04.1990
(51) Int. Cl.: C07K 3/20, C12N 9/64

(54) **PROCEDE DE PREPARATION DE LA PROTEINE C ACTIVEE**
VERFAHREN ZUR HERSTELLUNG VON AKTIVIERTEM PROTEIN C
PROCESS FOR PREPARATION OF ACTIVATED PROTEIN C

(30) Priorité: 12.04.1989 FR 8904814; 20.02.1990 FR 9002028
(43) Date de publication de la demande: 02.05.1991
(73) Titulaire: FONDATION NATIONALE DE TRANSFUSION SANGUINE, 75739 Paris Cédex 15 (FR)
(72) Inventeur: STEINBUCH, Marion, F-78960 Voisin-le-Bretonneux (FR); CHABBAT, Jacques, F-75017 Paris (FR); TABY, Olivier, F-92160 Antony (FR)
(74) Mandataire: Warcoin, Jacques
(86) Numéro de dépôt international: FR9000258
(87) Numéro de publication internationale: WO9012028

(56) Documents cités:
- EP-A- 0 287 028
- WO-A-85/00521

## Description

La présente invention concerne un procédé de préparation de la protéine C activée (APC).

La protéine C est une glycoprotéine plasmatique dont la forme active est une sérine protéase (1,2).

La protéine C humaine est une protéine de poids moléculaire 62 000 constituée de deux chaînes. Une chaîne lourde de poids moléculaire 41 000 portant le site actif et une chaîne légère de poids moléculaire 21 000, liées par un pont disulfure (2). Sa concentration plasmatique est de 3-5 mg/l.

Comme d'autres protéines, en particulier plasmatiques, cette protéine est un facteur vitamine K dépendant. Elle est synthétisée par le foie sous forme d'un précurseur. Les 11 premiers acides glutamiques de la chaîne légère sont carboxylés en position gamma par une carboxylase hépatique ayant une vitamine K pour cofacteur (3). Ces résidus gamma-carboxyglutamiques sont impliqués dans l'interaction avec l'ion calcium (4). Les phospholipides étant chargés négativement, le calcium réalise un pont ionique entre ceux-ci et la région appropriée des facteurs vitamine K dépendants. La chaîne légère possède par ailleurs un résidu béta-hydroxy-aspartique qui serait également impliqué dans l'intéraction avec le Ca⁺⁺.

Le proenzyme est transformé en protéine C activée (APC) par une coupure d'un dodécapeptide dans la partie N-terminale de la chaîne lourde (2). Cette réaction, qui se produit dans la microcirculation, est catalysée en présence de calcium par un complexe stoechiométrique 1:1 formé entre la thrombine et une protéine située à la surface des cellules endothéliales, la thrombomoduline (5).

La forme active a une action anticoagulante en inactivant les cofacteurs de la cascade de coagulation V et VIII par protéolyse limitée (6). L'enzyme actif augmenterait aussi la fibrinolyse en inactivant l'inhibiteur de l'activateur tissulaire du plasminogène.

L'APC n'exerce pleinement son activité qu'en présence d'un cofacteur, la protéine S, de phospholipides et de calcium. La protéine S est également une glycoprotéine plasmatique vitamine K dépendante. Elle est monocaténaire, de PM 75000, sa concentration plasmatique étant de 25 mg/l. Elle circule à 50 % sous forme libre et à 50 % sous forme de complexe non covalent avec une protéine du système du complément la "C4-Binding Protéin" (C4BP) (7). Lorsque la protéine S est liée au C4BP elle ne peut servir de cofacteur (7).

Contrairement aux autres enzymes vitamines K dépendants, la protéine C est anticoagulante.

On connait des déficits congénitaux et acquis en protéine C. On observe dans ce cas comme pour les déficits en AT III une tendance aux accidents thrombotiques répétés.

L'utilisation thérapeutique de l'enzyme et du proenzyme est intéressante. Ainsi, la protéine C peut être utilisée pour le traitement des déficits en protéine C et en particulier chez les nouveaux nés homozygotes qui développent un Purpura Fulminans.

Cette maladie, souvent léthale, est caractérisée par des lésions thrombotiques importantes qui nécessitent une thérapie par des concentrés plasmatiques enrichis en protéine C. La forme hétérozygote peut entraîner des épisodes thrombotiques légers à sévères ou être totalement asymptomatique.

La protéine C ainsi que la protéine C activée peuvent être employées pour la prophylaxie et le traitement des thromboses veineuses et artérielles, embolies pulmonaires cérébrales et cardiaques, CIVD, chocs septiques.

La protéine C activée peut être également utilisée pour le remplacement des anticoagulants classiques (en particulier l'héparine) sans leurs effets secondaires lors de thromboses artérielles ou veineuses, d'interventions chirurgicales, de phlébites, et dans le cas de réoclusion des infarctus du myocarde.

Il existe une grande homologie structurale entre certains facteurs vitamines K dépendants et en particulier entre les facteurs VII, IX, X et protéine C. Ils ont de plus un poids moléculaire très voisin (respectivement de 56 000, 57 000, 59 000, 62 000).

La similitude de ces caractéristiques moléculaires pose donc des difficultés dans l'isolement de ces différents facteurs.

La présente invention a précisément pour but de surmonter cet inconvénient et de proposer un procédé permettant d'obtenir rapidement la protéine C activée à partir d'une fraction sanguine plus ou moins enrichie en protéine C.

Il a en effet été mis en évidence par les inventeurs que l'aprotinine qui est un polypeptide d'origine animale de 58 acides aminés inhibe l'APC. L'intéraction étant réversible, il a été possible de purifier l'APC sur aprotinine insolubilisée.

Plus particulièrement, il s'agit d'un procédé de préparation de protéine C activée à partir d'une matière première contenant ladite protéine activée, caractérisé en ce qu'on adsorbe la protéine C activée sur de l'aprotinine insolubilisée puis qu'après lavage dudit complexe APC-aprotinine avec une solution tamponnée saline, ladite protéine C activée est recueillie par élution avec une solution aqueuse acide ou une solution d'un agent chaotrope.

La matière première contenant de la protéine C peut provenir d'origines diverses. Il peut s'agir de différentes fractions obtenues par les technologies de fractionnement sanguin, par exemple il peut s'agir de la fraction dite PPSB, ou bien de fractions telles que le prééluat de PPSB, des fractions obtenues lors du fractionnement à l'éthanol, ou bien des fractions obtenues par purification préalable, soit par immunopurification comme cela est par exemple décrit dans le brevet européen n^{o} 138 222, ou bien des fractions enrichies en protéine C tels qu'elles sont décrites par exemple dans British Journal of Haematology, 1988, 70, 436-440.

Selon un mode de réalisation particulier de l'invention, la matière première est un concentré de complexe prothrombique. La protéine C présente dans ce concentré y est activée par de la thrombine que l'on génère dans ladite matière première, par addition de calcium, ce calcium étant par la suite éliminé de manière à permettre l'activation de la protéine C par la thrombine générée in situ. Dans ce cas, le calcium est ajouté à la matière première, à une concentration comprise entre 10 et 100 mM, puis en est éliminé par dialyse ou diafiltration.

La fixation de l'APC est conduite de préférence à un pH compris entre 7 et 9, en général à un pH optimum de 8 à 8,4 avec une force ionique appropriée mais ne dépassant pas 0,4 M NaCl et de préférence inférieure à 0,25 M NaCl.

Le complexe ainsi formé entre l'aprotinine et l'APC est suffisamment stable, pour être soumis à une préélution avec des solutions salines sans que l'APC soit décrochée.

Pour éluer l'APC, il convient de prendre des conditions d'élution dissociant le complexe, notamment des pH extrêmes, en particulier des pH compris entre 1 et 3. La protéine C activée peut être également recueillie par élution avec une solution contenant un agent chaotrope par exemple le KSCN ou le NaSCN. L'agent chaotrope sera ensuite éliminé par dialyse.

La réaction de complexation est très spécifique et le produit élué est exempt de facteur II, de facteur VII, de facteur IX et de facteur X, ceci lorsque l'on part de la fraction PPSB et ceci avec un rendement très satisfaisant.

Comme cela a été indiqué précédemment, la fraction traitée doit contenir la protéine C sous forme activée, cette activation peut être effectuée de différentes façons, soit en utilisant la thrombine, ou bien en utilisant l'activateur spécifique de la protéine C extrait de venin de serpent, ou bien l'activateur naturel constitué de thrombine et de thrombomoduline.

Certaines fractions pourront déjà contenir la protéine C sous forme activée, dans ce cas l'activation ne sera bien évidemment pas nécessaire.

Les exemples ci-après sont destinés à mettre en évidence d'autres avantages et caractéristiques de la présente invention.

### EXEMPLE 1 :

La matière première utilisée pour cet exemple est une protéine C obtenue par immunopurification.

### Immunopurification de la protéine C :

Une sous fraction du plasma enrichie en protéine C est adsorbée sur une colonne d'anticorps monoclonaux anti-protéine C insolubilisés sur du Sepharose 4-B activé au bromure de cyanogène (Pharmacia). L'anticorps monoclonal employé reconnait la protéine C et ne reconnait pas l'APC.

Une préélution est effectuée avec un tampon Tris-HCl 50 mM pH 8, NaCl 0,5 M. L'élution est réalisée avec une solution 3 M thiocyanate de potassium. L'éluat obtenu est concentré et dialysé jusqu'à 75 mM NaCl tamponné à pH 8.

On obtient ainsi une solution de protéine C et aussi bien le rapport antigène sur concentration protéique que l'électrophorèse en SDS PAGE montrent un haut degré de purification.

Le proenzyme est activé par un des activateurs connus : thrombine-thrombomoduline, thrombine, extraits de venin d'Agkistrodon Contortrix ou de Russell's Viper. Ces activateurs peuvent être ajoutés dans la fraction en solution ou insolubilisés sur une matrice. Dans ce dernier cas, l'activation se fait en batch ou sur une colonne.

2 ml de protéine C immunopurifiée activée sont déposées sur une colonne (1 x 5) d'aprotinine-Sepharose 4-B (Pharmacia) (5 mg d'aprotinine par ml de gel) équilibrée par un tampon Tris-HCl 50 mM pH 8, NaCl 75 mM.

Une préélution est réalisée avec un tampon Tris-HCl 50 mM pH 8, NaCl 0,5 M.

L'APC est éluée en abaissant le pH avec une solution d'HCl 0,1 N et le pH est aussitôt remonté à 8.

Le dosage amidolytique, l'activité coagulante ainsi que l'immunoélectrophorèse unidimensionnelle Laurell anti protéine C montrent que l'on retrouve de la protéine C activée uniquement dans l'éluat HCl.

### EXEMPLE 2 :

La matière première utilisée pour ce deuxième exemple est un concentré du complexe prothrombique (PPSB). La protéine C contenue dans cette préparation est activée par un activateur comme pour l'exemple 1.

100 ml de matière première sont déposés sur une colonne (1 x 5) d'aprotinine-Sepharose 4-B ( Pharmacia ) (5 mg d'aprotinine par ml de gel) équilibrée par un tampon Tris-HCL 50 mM pH 8, NaCl 0,15 M.

Une préélution est effectuée avec un tampon Tris-HCl 50 mM pH 8, NaCl 0,5 M.

Aucune activité protéine C activée n'est décelable dans la fraction non retenue et dans la préélution 0,5 M NaCl.

L'APC spécifiquement retenue sur la matrice est éluée en abaissant le pH avec une solution d'HCl 0,1 N. Le pH est immédiatement remonté à 8.

La fraction éluée présente les caractéristiques de l'APC tant sur le point activité biologique que du comportement électrophorétique et de l'antigénicité. Les techniques utilisées sont respectivement dosages des activités anticoagulante et amidolytique, identification immunologique et électrophorèse SDS PAGE avec et sans réducteur.

### EXEMPLE 3 :

Du calcium (25 mM final) est ajouté à la fraction dite PPSB d'un concentré du complexe prothrombique. Après agitation douce pendant 30 minutes à 4 heures, le calcium est éliminé par dialyse ou diafiltration contre un tampon contenant par exemple 50 mM Tris-HCl; 0,15 M NaCl pH 7,4.

Par la suite, l'activation de la protéine C s'effectue sous agitation douce à température ambiante ou à 4°C pendant 8 à 24 heures.

La fraction ainsi obtenue est déposée sur aprotinine-Sépharose afin de purifier la protéine C activée.

Cette purification de la protéine C activée est réalisée selon le procédé décrit précédemment.

### BIBLIOGRAPHIE

1) Stenflo J., (1976), J. Biol. Chem. 251, 355-363
2) Kisiel W., (1979), J. Clin. Invest. 64, 761-769
3) Ferlund P., Stenflo J. (1982), J. Biol. Chem. 257,12170-12179
4) Nelsestuen G.L., Kisiel W., Di Scipio R.G., (1978), biochemistry 17,2134-2138
5) Esmon C.T., Owen W.G., (1981), Proc. Natl. Acad. Sci. USA. 78,2249-2252
6) Walker F.J., Sexton P.W., Esmon C.T., (1979), Biochim Biophys. Acta 571,333-342
7) Dahlbäck B., (1986) J. Biol. Chem. 261,12022-12027

## Revendications

1. Procédé de préparation de protéine C (APC) activée à partir d'une matière première contenant ladite protéine C activée, caractérisé en ce qu'on adsorbe l'APC sur de l'aprotinine insolubilisée puis qu'après lavage du dit complexe APC-aprotinine avec une solution tamponnée saline, ladite protéine C activée est recueillie par élution avec une solution aqueuse acide ou une solution d'un agent chaotrope.

2. Procédé selon la revendication 1, caractérisé en ce que la fixation de la protéine C activée sur l'aprotinine insolubilisée est réalisée à un pH compris entre 7 et 9 et de préférence entre 8 et 8,4.

3. Procédé selon la revendication 1 ou 2, caractérisé en ce que la protéine C activée est recueillie par élution avec une solution aqueuse acide de pH situé entre 1 et 3.

4. Procédé selon la revendication 1 ou 2, caractérisé en ce que la protéine C activée est recueillie par élution avec une solution contenant un agent chaïotrope choisi de préférence parmi le KSCN ou le NaSCN, ledit agent chaotrope étant ensuite éliminé par dialyse.

5. Procédé selon l'une des revendications 1 à 4, caractérisé en ce que le lavage du complexe protéine C activée-aprotinine est effectué avec une solution tamponnée saline de pH de l'ordre de 8.

6. Procédé selon l'une des revendications 1 à 5, caractérisé en ce que l'aprotinine est insolubilisée sur une matrice à des concentrations comprises entre 0,5 et 5 mg par ml de gel.

7. Procédé selon l'une des revendications 1 à 6, caractérisé en ce que la matière première contenant ladite APC est choisie parmi :
- la protéine C immunopurifiée activée et,
- le concentré du complexe prothrombique (PPSB) traité par un activateur de la protéine C.

8. Procédé selon l'une des revendications 1 à 6 caractérisée en ce que la matière première est un concentré du complexe prothrombique et, en ce que la protéine C y est activée par de la thrombine générée dans ladite matière première par addition de calcium, ledit calcium étant par la suite éliminé de manière à permettre l'activation de la protéine C par ladite thrombine.

9. Procédé selon la revendication 8, caractérisé en ce que le calcium est ajouté à la matière première, à une concentration comprise entre 10 et 100 mM.

## Claims

1. Method for preparing activated protein C (APC) from a starting material containing the said activated protein C, characterized in that the APC is adsorbed on insolubilized aprotinin and then in that, after washing of the said APC/aprotinin complex with a buffered saline solution, the said activated protein C is collected by elution with an acidic aqueous solution or a solution of a chaotropic agent.

2. Method according to Claim 1, characterized in that the binding of the activated protein C to the insolubilized aprotinin is carried out at a pH of between 7 and 9, and preferably between 8 and 8.4.

3. Method according to Claim 1 or 2, characterized in that the activated protein C is collected by elution with an acidic aqueous solution having a pH lying between 1 and 3.

4. Method according to Claim 1 or 2, characterized in that the activated protein C is collected by elution with a solution containing a chaotropic agent preferably selected from KSCN or NaSCN, the said chaotropic agent then being removed by dialysis.

5. Method according to one of Claims 1 to 4, characterized in that the washing of the activated protein C/aprotinin complex is performed with a buffered saline solution having a pH of the order of 8.

6. Method according to one of Claims 1 to 5, characterized in that the aprotinin is insolubilized on a matrix at concentrations of between 0.5 and 5 mg per ml of gel.

7. Method according to one of Claims 1 to 6, characterized in that the starting material containing the said APC is selected from:
- activated immunopurified protein C, and
- concentrate of the prothrombin complex (PPSB) treated with an activator of protein C.

8. Method according to one of Claims 1 to 6, characterized in that the starting material is a concentrate of the prothrombin complex, and in that the protein C is activated therein by thrombin generated in the said starting material by adding calcium, the said calcium being subsequently removed so as to permit activation of the protein C by the said thrombin.

9. Method according to Claim 8, characterized in that the calcium is added to the starting material at a concentration of between 10 and 100 mM.

## Patentansprüche

1. Verfahren zur Herstellung von aktiviertem Protein C (APC) aus einem Ausgangsmaterial, welches das genannte aktivierte Protein C enthält, dadurch gekennzeichnet, daß man das APC an insolubilisiertem Aprotinin adsorbiert und dann nach dem Waschen des genannten APC-Aprotinin-Komplexes mit einer gepufferten Salzlösung das genannte aktivierte Protein C durch Elution mit einer sauren wäßrigen Lösung oder mit einer Lösung eines chaotropen Agens gewinnt (abtrennt).

2. Verfahren nach Anspruch 1, dadurch gekennzeichnet, daß die Fixierung des aktivierten Proteins C an dem insolubilisierten Aprotinin bei einem pH-Wert zwischen 7 und 9 und vorzugsweise zwischen 8 und 8,4 durchgeführt wird.

3. Verfahren nach Anspruch 1 oder 2, dadurch gekennzeichnet, daß das aktivierte Protein C durch Eluieren mit einer sauren wäßrigen Lösung mit einem pH-Wert zwischen 1 und 3 gewonnen (abgetrennt) wird.

4. Verfahren nach Anspruch 1 oder 2, dadurch gekennzeichnet, daß das aktivierte Protein C durch Eluieren mit einer Lösung, die ein chaotropes Agens enthält, das vorzugsweise ausgewählt wird aus der Gruppe KSCN und NaSCN, gewonnen (abgetrennt) wird, wobei das chaotrope Agens anschließend durch Dialyse eliminiert wird.

5. Verfahren nach einem der Ansprüche 1 bis 4, dadurch gekennzeichnet, daß das Waschen des aktivierten Protein C-Aprotinin-Komplexes mit einer gepufferten Salzlösung mit einem pH-Wert in der Größenordnung von 8 durchgeführt wird.

6. Verfahren nach einem der Ansprüche 1 bis 5, dadurch gekennzeichnet, daß das Aprotinin auf einer Matrix in Konzentrationen zwischen 0,5 und 5 mg pro ml Gel insolubilisiert wird.

7. Verfahren nach einem der Ansprüche 1 bis 6, dadurch gekennzeichnet, daß das Ausgangsmaterial, welches das APC enthält, ausgewählt wird aus der Gruppe
- immungereinigtes aktiviertes Protein C und
- mit einem Aktivator für das Protein C behandeltes Konzentrat des Prothrombin-Komplexes (PPSB).

8. Verfahren nach einem der Ansprüche 1 bis 6, dadurch gekennzeichnet, daß das Ausgangsmaterial ein Konzentrat des Prothrombin-Komplexes ist und daß das Protein C darin durch das in dem Ausgangsmaterial durch Zugabe von Calcium erzeugte Thrombin aktiviert worden ist, wobei das Calcium anschließend so eliminiert wird, daß die Aktivierung des Proteins C durch das genannte Thrombin möglich ist.

9. Verfahren nach Anspruch 8, dadurch gekennzeichnet, daß das Calcium dem Ausgangsmaterial in einer Konzentration zwischen 10 und 100 mM zugesetzt wird.
